# EUROPÄISCHE PATENTSCHRIFT

(11) **EP 0 786 449 B1**
(45) Veröffentlichungstag und Bekanntmachung des Hinweises auf die Patenterteilung: **27.10.1999**
(21) Anmeldenummer: 97100780.2
(22) Anmeldetag: 20.01.1997
(51) Int. Cl.: C07C 209/60

(54) **Verfahren zur Herstellung von Aminen aus Olefinen an hexagonalen Faujasiten**
Process for the preparation of amines from olefins on hexagonal faujasites
Procédé de préparation d'amines à partir d'oléfines utilisant des faujasites hexagonals

(30) Priorität: 26.01.1996 DE 19602709
(43) Veröffentlichungstag der Anmeldung: 30.07.1997
(73) Patentinhaber: BASF AKTIENGESELLSCHAFT, 67056 Ludwigshafen (DE)
(72) Erfinder: Eller, Karsten, Dr., 67059 Ludwigshafen (DE); Kummer, Rudolf, Dr., 67227 Frankenthal (DE); Gehrer, Eugen, Dr., 67069 Ludwigshafen (DE)

(56) Entgegenhaltungen:
- EP-A- 0 039 918
- EP-A- 0 305 564
- EP-A- 0 587 424
- US-A- 5 273 945

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von Zeolithen des Typs EMT.

Eine Übersicht über die Methoden zur Aminierung von Olefinen wird in "Functionalisation of Alkenes: Catalytic Amination of Monoolefins", J.J. Brunet et al. J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 536 602, EP-A-101 921 oder DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Aus EP-A-39 918 ist ein Verfahren bekannt, um Amine aus Olefinen an verschiedenen Faujasiten vom Typ Y- oder X-Zeolith herzustellen. Die erhaltenen Umsätze, z.B. im Falle von Isobuten maximal 6,5%, sind jedoch sehr niedrig.

Auch EP-A-305 564 benutzt Faujasite vom Typ Y, zum Teil in dealuminierter Form, für Aminierungsreaktionen. Auch hier werden mit Isobuten nur maximal 7,8% Umsatz erzielt.

In der EP-A-587 424 werden mit dealuminierten Y-Zeolithen zwar höhere Umsätze erreicht (max. 17,1%), allerdings sind die verwendeten Belastungen niedrig, so daß die Raum-Zeit-Ausbeuten für eine technische Verwendung zu gering sind.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Überraschend wurde nun gefunden, daß bei Verwendung von hexagonalen Faujasiten (EMT) anstelle der bisher verwendeten kubischen Faujasite (FAU) den oben genannten Nachteilen abgeholfen werden kann.

Demgemäß wurde ein neues und verbessertes Verfahren zur Herstellung von Aminen der allgemeinen Formel I 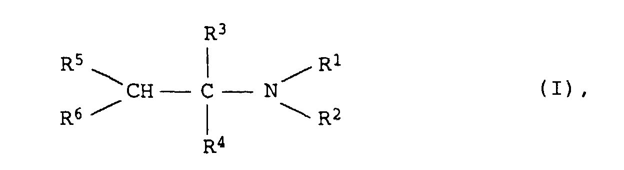 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam eine gesättigte oder ungesättigte C₃-bis C₉-Alkylendikette und
- R³ oder R⁵: C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 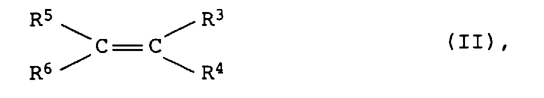 in der R³, R⁴, R⁵ und R⁶ die oben genannten Bedeutungen haben, mit Ammoniak oder primären oder sekundären Aminen der allgemeinen Formel III 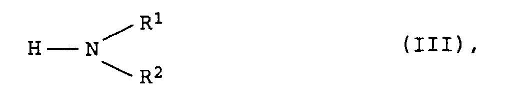 in der R¹ und R² die oben genannten Bedeutungen haben, bei Temperaturen von 200 bis 350°C und einem Druck von 100 bis 300 bar in Gegenwart eines Heterogenkatalysators gefunden, welches dadurch gekennzeichnet ist, daß man hexagonalen Faujasit enthaltende Heterogenkatalysatoren einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von hexagonalen Faujasiten als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Beispielsweise kann man als Olefin II Isobuten, Diisobuten, Cyclopenten, Cyclohexen oder Polyisobuten einsetzen. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich hexagonale Faujasite (EMT) oder deren Mischkristalle mit kubischem Faujasit (EMT-FAU). Derartige Materialien sind beispielsweise aus Zeolites 11 (1991), S. 98 bekannt und werden auch als EMC-2 (EMT) bezeichnet; eine andere Bezeichnung ist Breck Structure Six (BSS). EMT-FAU-Intermediate sind als CSZ-1 aus der US-A-4 309 313, CSZ-3 aus der US-A-4 333 859, ECR-4 aus der US-A-4 714 601, ECR-17 aus der EP-A-259 526, ECR-30 aus der EP-A-315 461, ECR-32, LZ-267 aus der US-A-4 503 023, ZSM-3 aus der US-A-3 415 736 oder ZSM-20 aus der US-A-3 972 983 bekannt.

Die erfindungsgemäßen hexagonalen Faujasite können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 h getrocknet und bei 200 bis 600°C/2 bis 16 h calciniert, wobei die Calcinierung auch direkt (in situ) im Aminierungsreaktor erfolgen kann.

Zur Erhöhung der Selektivität, der Standzeit und der Anzahl der möglichen Regenerierungen kann man verschiedene Modifizierungen an den erfindungsgemäßen hexagonalen Faujasiten vornehmen.

Eine Modifizierung der Katalysatoren besteht darin, daß man die unverformten oder die verformten hexagonalen Faujasite mit Alkalimetallen wie Na und K, Erdalkalimetallen wie Ca, Mg, Erdmetallen wie Tl, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen hexagonalen Faujasite in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium-und Alkaliform der erfindungsgemäßen hexagonalen Faujasite vorgenommen werden.

Eine weitere Möglichkeit der Metallaufbringung auf die erfindungsgemäßen hexagonalen Faujasite besteht darin, daß man das Material z.B. mit einem Halogenid, einem Nitrat, einem Acetat, einem Oxalat, einem Citrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten hexagonalen Faujasiten kann eine Nachbehandlung mit Wasserstoff und/oder mit Wasserdampf günstig sein.

Eine weitere Möglichkeit der Modifizierung besteht darin, daß man die erfindungsgemäßen hexagonalen Faujasite - verformt oder unverformt - einer Behandlung mit Säuren, wie Salzsäure (HCl), Flußsäure (HF), Schwefelsäure (H₂SO₄), Oxalsäure (HO₂C-CO₂H), Phosphorsäure (H₃PO₄) oder deren Gemischen unterwirft.

Eine besondere Ausführungsform besteht darin, daß man die erfindungsgemäßen hexagonalen Faujasite vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen hexagonalen Faujasite nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung wieder direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen hexagonalen Faujasiten vorgenommen werden kann, ist eine Dealuminierung, bei der ein Teil der Aluminiumatome durch Silicium ersetzt wird oder die Zeolithe durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987) Seiten 495 bis 503.

Die Katalysatoren kann man als Stränge mit Durchmessern von z.B. 1 bis 4 mm oder als Tabletten mit z.B. 3 bis 5 mm Durchmesser für die Aminierung der Olefine einsetzen.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹, R², R³, R⁴, R⁵, R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₈-Alkinyl, insbesondere C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
   - C₄- bis C₂₀-Cycloalkyl-alkyl, -alkyl, bevorzugt C₄- bis C₁₂-Cycloalkyl-alkyl, besonders bevorzugt C₅- bis C₁₀-Cycloalkyl-alkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
R¹ und R²
   - gemeinsam eine gesättigte oder ungesättigte C₃- bis C₉-Alkylendikette, bevorzugt -(CH₂)₄-, -(CH₂)₅-, -(CH₂)₇- und -CH=CH-CH=CH-,
R³ oder R⁵
   - C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
   - gemeinsam eine C₂- bis C₁₂-Alkylendikette, bevorzugt eine C₃-bis C₈-Alkylendikette, besonders bevorzugt -(CH₂)₃-, -(CH₂)₄-, -(CH₂)₅-, -(CH₂)₆- und - (CH₂)₇-, insbesondere -(CH₂)₃- und -(CH₂)₄-.

### Beispiele

### Katalysatorsynthesen

### Katalysator A: Herstellung von H-EMT

80 g Na-EMT mit einem SiO₂/Al₂O₃-Verhältnis von 7:1 wurde mit 1200 g einer 20 %-iger NH₄Cl-Lösung bei 80°C ausgetauscht und anschließend mit 2 Liter Wasser gewaschen. Nach erneutem NH₄Cl-Austausch und Nachwaschen wurde der Zeolith 2 Stunden bei 120°C getrocknet und 5 Stunden bei 500°C calciniert. Der gesamte Vorgang wurde noch einmal wiederholt und der Na-Gehalt des Zeolithen vor der letzten Calcinierung mit 0,03 Gew.-% bestimmt.

65 g H-EMT wurden mit 43 g Boehmit und 2,2 g Ameisensäure im Kneter kompaktiert und unter Wasserzusatz (68 ml) 60 min verknetet. In einer Strangpresse wurden mit einem Pressdruck von 90 bar 2 mm Stränge erzeugt und diese 4 Stunden bei 120°C getrocknet und 16 Stunden bei 500°C calciniert.

### Katalysator B: Herstellung eines HY-Zeolithen (Vergleichsbeispiel)

2160 g NaY wurden mit 1440 g Boehmit und 72 g Ameisensäure versetzt, im Kneter kompaktiert und unter Wasserzusatz (1850 ml) 60 min verknetet. In einer Strangpresse wurden mit einem Pressdruck von 90 bar 2 mm Stränge erzeugt und diese 16 Stunden bei 110°C getrocknet und 16 Stunden bei 500°C calciniert. Die fertigen Stränge wurden analog zu Katalysator A viermal mit 20 %-iger NH₄Cl-Lösung bei 80°C ionengetauscht und schließlich 5 Stunden bei 500°C calciniert.

### Katalysator C: Herstellung eines HX-Zeolithen (Vergleichsbeispiel)

13X-Stränge der Firma Union Carbide wurden analog zu Katalysator A viermal mit 20 %-iger NH₄Cl-Lösung bei 80°C ionengetauscht und enthielten nach der Calcinierung noch 0,67 % Natrium.

### Katalysator D: Herstellung eines USY-Zeolithen (Vergleichsbeispiel)

180 g eines dealuminierten Y-Zeolithen der Firma Grace (USY) wurden mit 120 g Boehmit und 6 g Ameisensäure versetzt, im Kneter kompaktiert und unter Wasserzusatz (210 ml) 45 min verknetet. In einer Strangpresse wurden mit einem Pressdruck von 80 bar 2 mm Stränge erzeugt und diese 16 Stunden bei 110°C getrocknet und 16 Stunden bei 500°C calciniert.

### Katalysator E: Herstellung eines LaY-Zeolithen (Vergleichsbeispiel)

Na-Y Stränge aus Beispiel B wurden zweimal mit wäßriger La(NO₃)₃-Lösung ausgetauscht. Nach Analyse enthielt der fertige Katalysator 6,85 Gew.-% Lanthan und 1,5 Gew.-% Natrium.

### Katalysator F: Herstellung eines dealuminierten EMT-Zeolithen

20 g Katalysator A wurden in ein Drehrohr eingebaut und 2 Stunden bei 80°C mit Stickstoff (10 l•h⁻¹) getrocknet. Nach Abkühlung auf Raumtemperatur wurde dem N₂-Strom über einen Sättiger SiCl₄ zugesetzt und die Temperatur innerhalb von 25 min auf 460°C erhöht, 2 Stunden gehalten und dann die SiCl₄-Zufuhr abgestellt und unter Stickstoff auf Raumtemperatur abgekühlt. Der ausgebaute Katalysator wurde dann an Luft 5 Stunden bei 500°C calciniert. Der Aluminiumgehalt sank durch die Dealuminierung von 22,3 Gew.-% (Katalysator A) auf 18,8 Gew.-%.

### Aminierungsbeispiele

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis von 1:1,5 durchgeführt. Die Reaktionsprodukte wurden im Gaschromatographen analysiert.

Die Ergebnisse sind in Tabelle 1 zusammengestellt und zeigen, daß die erfindungsgemäßen Katalysatoren höhere Ausbeuten als die bisher bekannten Katalysatorsysteme auf Basis von Faujasiten (H-X, H-Y) aufweisen, und daß die Ausbeuten weiterhin auch über denen modifizierter Faujasite (dealuminierter Y, La-ausgetauschter Y) liegen.

**Tabelle 1:**

| tert.-Butylamin (NH₃ : C₄H₈ = 1,5) | | | | | | | |
|---|---|---|---|---|---|---|---|
| Katalysator | | Druck | Temperatur | tert.-Butylamin-Ausbeute [Gew.-%] | | | Litergewicht |
| Nr. | Al₂O₃ [Gew.-%] | [bar] | [°C] | WHSV 0,7 [g/g·h] | WHSV 1,5 [g/g·h] | WHSV 3 [g/g·h] | [kg/l] |
| A | 40 | 280 | 260 | 18,10 | 11,80 | | 0,50 |
| A | 40 | 280 | 270 | 20,02 | 16,53 | 12,12 | 0,50 |
| A | 40 | 280 | 280 | 17,55 | 17,14 | 15,19 | 0,50 |
| A | 40 | 280 | 300 | 12,41 | 12,76 | 12,36 | 0,50 |
| B | 40 | 280 | 270 | 19,12 | 11,50 | 6,16 | 0,63 |
| C | | 280 | 300 | 7,57 | 4,96 | 3,10 | 0,56 |
| D | 40 | 280 | 270 | 12,36 | 8,21 | 5,08 | 0,50 |
| E | 40 | 280 | 270 | 16,50 | 11,40 | 7,52 | 0,67 |
| F | | 280 | 270 | 17,52 | 13,23 | 9,18 | 0,48 |
| WHSV = Weight hourly space velocity (g Edukte pro g Katalysator und pro Stunde) | | | | | | | |

## Patentansprüche

1. Verfahren zur Herstellung von Aminen der allgemeinen Formel I 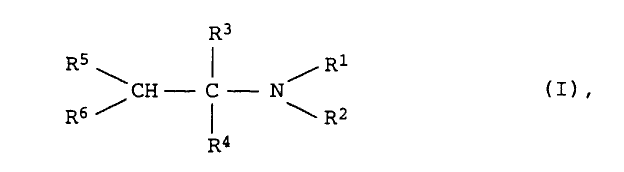 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C20-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
R¹ und R² gemeinsam eine gesättigte oder ungesättigte C₃- bis C₉-Alkylendikette und
R³ oder R⁵ C₂₁- bis C₂₀₀-Alkyl, C₂₁- bis C₂₀₀-Alkenyl oder gemeinsam eine C₂- bis C₁₂-Alkylendikette
bedeuten, durch Umsetzung von Olefinen der allgemeinen Formel II 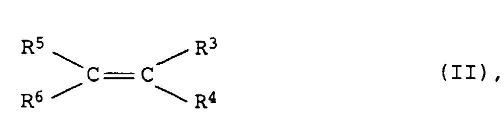 in der R³, R⁴, R⁵ und R⁶ die oben genannten Bedeutungen haben, mit Ammoniak oder primären oder sekundären Aminen der allgemeinen Formel III 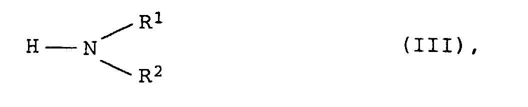 in der R¹ und R² die oben genannten Bedeutungen haben, bei Temperaturen von 200 bis 350°C und einem Druck von 100 bis 300 bar in Gegenwart eines Heterogenkatalysators, dadurch gekennzeichnet, daß man hexagonalen Faujasit enthaltende Heterogenkatalysatoren einsetzt.

2. Verfahren zur Herstellung von Aminen I nach Anspruch 1, dadurch gekennzeichnet, daß man das gebildete Amin I abtrennt und die nichtumgesetzten Einsatzstoffe II und III zurückführt.

3. Verfahren zur Herstellung von Aminen nach den Ansprüchen 1 bis 2, dadurch gekennzeichnet, daß man als Olefin II Isobuten, Diisobuten, Cyclopenten, Cyclohexen oder Polyisobuten einsetzt.

4. Verfahren zur Herstellung von Aminen nach den Ansprüchen 1 bis 3, dadurch gekennzeichnet, daß man dealuminierten hexagonalen Faujasit enthaltende Heterogenkatalysatoren einsetzt.

5. Verfahren zur Herstellung von Aminen nach den Ansprüchen 1 bis 4, dadurch gekennzeichnet, daß man hexagonalen Faujasit enthaltende Heterogenkatalysatoren in der H-Form oder in der Ammoniumform einsetzt.

6. Verfahren zur Herstellung von Aminen nach den Ansprüchen 1 bis 5, dadurch gekennzeichnet, daß man hexagonalen Faujasit enthaltende Heterogenkatalysatoren einsetzt, die mit einer Säure, insbesondere einer aus der Gruppe Salzsäure, Fluß-; säure, Schwefelsäure, Phosphorsäure, Oxalsäure oder deren Gemischen, behandelt sind.

7. Verfahren zur Herstellung von Aminen nach den Ansprüchen 1 bis 6, dadurch gekennzeichnet, daß man hexagonalen Faujasit enthaltende Heterogenkatalysatoren, die mit einem oder mehreren Übergangsmetallen dotiert sind, einsetzt.

8. Verfahren zur Herstellung von Aminen nach den Ansprüchen 1 bis 7, dadurch gekennzeichnet, daß man hexagonalen Faujasit enthaltende Heterogenkatalysatoren, die mit einem oder mehreren Elementen der Seltenen Erden dotiert sind, einsetzt.

9. Verfahren zur Herstellung von Aminen nach den Ansprüchen 1 bis 8, dadurch gekennzeichnet, daß man hexagonalen Faujasit enthaltende Heterogenkatalysatoren einsetzt, die im Reaktor (in situ) durch Calcinierung der Templat-haltigen Form erzeugt werden.

10. Verfahren zur Herstellung von Aminen nach den Ansprüchen 1 bis 9, dadurch gekennzeichnet, daß man hexagonalen Faujasit enthaltende Heterogenkatalysatoren einsetzt, die mit einem oder mehreren Elementen aus der Gruppe der Alkali-, Erdalkalimetalle oder Erdmetalle dotiert sind.

11. Verfahren zur Herstellung von Aminen nach den Ansprüchen 1 bis 10, dadurch gekennzeichnet, daß man hexagonalen Faujasit enthaltende Heterogenkatalysatoren einsetzt, die mit einem Bindemittel verformt und bei Temperaturen von 200 bis 600°C calciniert sind.

## Claims

1. A process for preparing amines of the general formula I 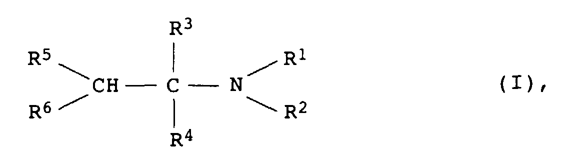 where
R¹,R²,R³,R⁴,R⁵ and R⁶ are each hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl,
R¹ and R² are together a saturated or unsaturated C₃-C₉-alkylene dichain, and
R³ or R⁵ is C₂₁-C₂₀₀-alkyl or C₂₁-C₂₀₀-alkenyl or together they are a C₂-C₁₂-alkylene dichain,
by reacting olefins of the general formula II 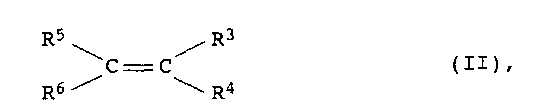 where R³, R⁴, R⁵ and R⁶ are each as defined above, with ammonia or primary or secondary amines of the general formula III 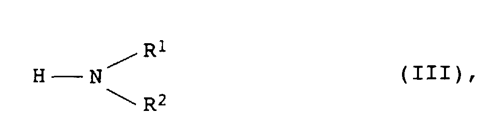 where R¹ and R² are each as defined above, at temperatures from 200 to 350°C and pressures from 100 to 300 bar in the presence of a heterogeneous catalyst, which comprises using a heterogeneous catalyst comprising hexagonal faujasite.

2. A process as claimed in claim 1, wherein the product amine I is separated off and the unconverted feed materials II and III are recycled.

3. A process as claimed in claim 1 or 2, wherein olefin II is isobutene, diisobutene, cyclopentene, cyclohexene or polyisobutene.

4. A process as claimed in any of claims 1 to 3, wherein the heterogeneous catalyst used comprises dealuminated hexagonal faujasite.

5. A process as claimed in any of claims 1 to 4, wherein the hexagonal faujasite heterogeneous catalyst used is in the H-form or in the ammonium form.

6. A process as claimed in any of claims 1 to 5, wherein the hexagonal faujasite heterogeneous catalyst used has been treated with an acid, especially with an acid selected from the group consisting of hydrochloric acid, hydrofluoric acid, sulfuric acid, phosphoric acid, oxalic acid and mixtures thereof.

7. A process as claimed in any of claims 1 to 6, wherein the hexagonal faujasite heterogeneous catalyst used has been doped with one or more transition metals.

8. A process as claimed in any of claims 1 to 7, wherein the hexagonal faujasite heterogeneous catalyst used has been doped with one or more rare earth elements.

9. A process as claimed in any of claims 1 to 8, wherein the hexagonal faujasite heterogeneous catalyst used is formed in situ in the reactor by calcination of the template-containing form.

10. A process as claimed in any of claims 1 to 9, wherein the hexagonal faujasite heterogeneous catalyst used has been doped with one or more elements from the group of the alkali, alkaline earth or earth metals.

11. A process as claimed in any of claims 1 to 10, wherein the hexagonal faujasite heterogeneous catalyst used has been molded with a binder and calcined at from 200 to 600°C.

## Revendications

1. Procédé pour la préparation d'amines de formule générale I 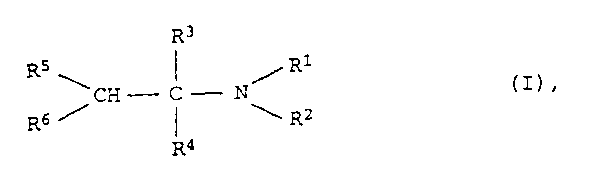 dans laquelle
R¹, R², R³, R⁴, R⁵, R⁶ représentent l'hydrogène, un groupe alkyle en C1-C20, alcényle en C2-C20, alcynyle en C2-C20, cycloalkyle en C3-C20, alkylcycloalkyle en C4-C20, cycloalkyl-alkyle en C4-C20, aryle, alkylaryle en C7-C20 ou aralkyle en C7-C20,
R¹ et R² représentent ensemble une chaîne divalente saturée ou insaturée alkylène en C3-C9 et
R³ ou R⁵ représentent un groupe alkyle en C21-C200, alcényle en C21-C200 ou représentent ensemble une chaîne divalente alkylène en C2-C12,
par réaction d'oléfines de formule générale II 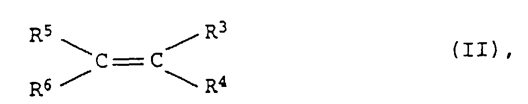 dans laquelle R³, R⁴, R⁵ et R⁶ ont les significations indiquées ci-dessus, avec l'ammoniac ou des amines primaires ou secondaires de formule générale III 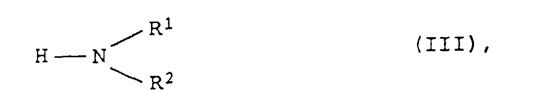 dans laquelle R¹ et R² ont les significations indiquées ci-dessus, à des températures de 200 à 350°C sous une pression de 100 à 300 bar en présence d'un catalyseur hétérogène, caractérisé par le fait que l'on utilise des catalyseurs hétérogènes contenant de la faujasite hexagonale.

2. Procédé pour la préparation des amines I selon la revendication 1, caractérisé par le fait que l'on sépare l'amine I formée et on recycle les composants de départ II et III non convertis.

3. Procédé pour la préparation d'amines selon les revendications 1 à 2, caractérisé par le fait que l'oléfine II mise en oeuvre est l'isobutène, le diisobutène, le cyclopentène, le cyclohexène ou un polyisobutène.

4. Procédé pour la préparation d'amines selon les revendications 1 à 3, caractérisé par le fait que l'on utilise des catalyseurs hétérogènes contenant une faujasite hexagonale désaluminisée.

5. Procédé pour la préparation d'amines selon les revendications 1 à 4, caractérisé par le fait que l'on utilise des catalyseurs hétérogènes contenant une faujasite hexagonale sous la forme H ou sous la forme ammonium.

6. Procédé pour la préparation d'amines selon les revendications 1 à 5, caractérisé par le fait que l'on utilise des catalyseurs hétérogènes contenant une faujasite hexagonale et qui ont été traités par un acide, en particulier un acide du groupe de l'acide chlorhydrique, de l'acide fluorhydrique, de l'acide sulfurique, de l'acide phosphorique, de l'acide oxalique ou leurs mélanges.

7. Procédé pour la préparation d'amines selon les revendications 1 à 6, caractérisé par le fait que l'on utilise des catalyseurs hétérogènes contenant une faujasite hexagonale et qui ont été dopés par un ou plusieurs métaux de transition.

8. Procédé pour la préparation d'amines selon les revendications 1 à 7, caractérisé par le fait que l'on utilise des catalyseurs hétérogènes contenant une faujasite hexagonale et qui ont été dopés par un ou plusieurs éléments des terres rares.

9. Procédé pour la préparation d'amines selon les revendications 1 à 8, caractérisé par le fait que l'on utilise des catalyseurs hétérogènes contenant une faujasite hexagonale et qui ont été formés dans le réacteur (in situ) par calcination de la forme comportant une matrice.

10. Procédé pour la préparation d'amines selon les revendications 1 à 9, caractérisé par le fait que l'on utilise des catalyseurs hétérogènes contenant une faujasite hexagonale et qui ont été dopés par un ou plusieurs éléments du groupe des métaux alcalins, des métaux alcalino-terreux ou des métaux terreux.

11. Procédé pour la préparation d'amines selon les revendications 1 à 10, caractérisé par le fait que l'on utilise des catalyseurs hétérogènes contenant une faujasite hexagonale, qui ont été façonnés avec un liant et calcinés à des températures de 200 à 600°C.
